# EUROPEAN PATENT SPECIFICATION

(11) **EP 3 886 600 B1**
(45) Date of publication and mention of the grant of the patent: **09.10.2024**
(21) Application number: 19927847.4
(22) Date of filing: 12.11.2019
(51) Int. Cl.: A23L 2/70, A23L 2/84, A23L 2/02, C12P 17/18

(54) **METHOD FOR ELIMINATING DEFECTS IN POMEGRANATE JUICE BY MEANS OF ENZYMATIC PROCESSES**
VERFAHREN ZUR BESEITIGUNG VON DEFEKTEN IN GRANATAPFELSAFT MITTELS ENZYMATISCHER VERFAHREN
PROCÉDÉ D'ÉLIMINATION DE DÉFAUTS D'UN JUS DE GRENADE PAR DES PROCÉDÉS ENZYMATIQUES

(30) Priority: 09.05.2019 TR 201906951
(43) Date of publication of application: 06.10.2021
(73) Proprietor: Ankara Üniversitesi, Çankaya/Ankara (TR)
(72) Inventor: TÜRKYILMAZ, Meltem, D kap Ankara (TR)
(74) Representative: Kaya, Erdem
(86) International application number: PCT/TR2019/000080
(87) International publication number: WO 2020/226578

(56) References cited:
- CN-A- 101 491 364
- CN-A- 103 960 714
- CN-A- 107 960 569
- FR-A1- 2 251 276
- US-A1- 2009 123 979
- US-A1- 2015 157 555
- CRISTINA GARCIA-MUNOZ, FABRICE VAILLANT: "Metabolic Fate of Ellagitannins: Implications for Health, and Research Perspectives for Innovative Functional Foods", CRITICAL REVIEWS IN FOOD SCIENCE AND NUTRITION, vol. 54, no. 12, 28 February 2014 (2014-02-28), pages 1584 - 1598, XP055605190
- MARTINA CERRETI ET AL.: "Optimization of Pectinase and Protease Clarification Treatment of Pomegranate Juice", LWT - FOOD SCIENCE AND TECHNOLOGY 82, vol. 82, 12 April 2017 (2017-04-12), pages 58 - 65, XP085018995
- ASHIMA KAPOOR, HINA IQBAL: "Efficiency of Tannase Produced by Trichoderma Harzianum MTCC 10841 in Pomegranate Juice Clarification and Natural Tannin Degradation", INTERNATIONAL JOURNAL OF LIFE SCIENCES BIOTECHNOLOGY AND PHARMA RESEARCH, vol. 4, no. 6, 2013, pages 641 - 650, XP055821406

## Description

### Technical Field of the Invention

This invention relates to a clarification process carried out by an enzymatic method involving the use of tannase, lactonase and protease for eliminating the defects (haze, sedimentation, acridity) and ensuring color stability in pomegranate juices.

### State of the Art

Color, degree of haze and sediment content of pomegranate juices are the most essential criteria affecting the user preference. Pomegranate juice products which are available in the market are known to be containing high degrees of sedimentation and their colors are far from being satisfactory in terms of consumer satisfaction. Today, color of pomegranate juice products may instantly be improved by means of physical methods such as ultrafiltration. However, formation of sediment in pomegranate juice products released to the market by companies using ultrafiltration method and the fact that these products are of an undesired brownish color, clearly indicated that the ultrafiltration method does not prevent sediment formation and fails to preserve the color of products during the storage period. In order to increase and popularize the consumption of pomegranate juice which is quite well established to be exceedingly beneficial to health, haze and sediment formation should be prevented and the stability of anthocyanins, during the storage period particularly, should be improved.

Until today, in addition to the ultrafiltration technique, various auxiliary clarification agents (polyvinylpolypyrrolidone (PVPP), gelatin, albumin, casein and chitosan) and effects produced by clarification methods (natural sedimentation, laccase, ultrafiltration and microfiltration) have been studied in order to solve the problem of haze and sediment formation in pomegranate juices.

In the entirety of these studies, it was considered that removing a specific amount of phenolics which are present in pomegranate juices would ensure clarification. However, in addition to failing in elimination of haze and sediment formation, a significant amount of anthocyanins which provide the bright red color to pomegranate juice were also removed therefrom.

In the prior art, it is known that amount of phenolic agent, hydrolysable tannin and anthocyanin decrease in the application of natural sedimentation carried out with use of gelatin, albumin and casein. Nevertheless, subsequent haze and acridity problems still persist.

While the total amount of phenolic agent is 1991 mg/L, hydrolysable tannin amount is 1418 mg/L as a result of natural sedimentation application in pomegranate juices. Whereas, as a result of the clarification process carried out with gelatin, casein and albumin, total phenolic agent contents of pomegranate juices decreased down to 1649, 739 and 1848 mg/L respectively. Hydrolyzed tannin contents respectively decreased to 932, 905 and 1247 mg/L. As a result of these clarification processes, gelatin (6.04 NTU) and casein (6.53 NTU) allowed for producing pomegranate juice of similar clarity, whereas use of albumin (10.10 NTU) resulted in a pomegranate juice product of lower clarity. Despite the fact that gelatin did manage to remove a greater amount of total phenolic agent from the pomegranate juice by 55% when compared to albumin, both of auxiliary clarification ingredients resulted in a similar reduction rate (34-36%) in terms of hydrolyzed tannin contents. Consequently, pomegranate juices with similar degrees of clarity were obtained. However, albumin use fell behind in terms of reducing both total phenolic agent amount and hydrolysable tannin amount when compared to gelatin and casein, and a pomegranate juice having a much higher degree of clarity (67%) was obtained. When all results are analyzed together, it can be clearly deduced that the phenolic agent group having an impact on clarity is hydrolysable tannins. However, abovementioned auxiliary clarification agents managed to reduce hydrolysable tannin amount by only 12-36%. A reduction in this rate was not enough for the clarification of the pomegranate juice.

In cases where chitosan and xanthan gum were utilized, total phenolic agent (respectively 1999 and 1992 mg/L) and hydrolysable tannins (respectively 1200 and 1296 mg/L) reduced in a very limited amount. However, a certain degree of clarity (respectively, 10.3 and 20.0 NTU) was achieved in pomegranate juices. This clarity is due to the reducing effect produced by chitosan and xanthan gum on proteins. However, protein reduction effect of these auxiliary clarification agents also failed to ensure the complete clarification of pomegranate juices.

In laccase and ultrafiltration applications, on the other hand, total phenolic agent amount was reduced by 50%. However, the reduction observed at this level failed to inhibit subsequent haze and sediment formation.

Haze and acridity problems that occur subsequently in pomegranate juices were not eliminated since hydrolyzed tannin and protein amounts could not be reduced at a desired level by means of prior methods. Moreover, differently from the present invention, bioavailability of the pomegranate juice also decreased since hydrolyzed tannin and protein were removed from the fruit's juice. In the present invention, however, these components will be transformed into forms that are utilized by human body for the purpose of reducing hydrolyzed tannin and protein amounts. Additionally, in the present invention, anthocyanin stability will also increase as substances created as a result of enzymatic decomposition of hydrolyzed tannin and proteins will produce a co-pigmentation effect.

Patent applications pertaining to clarification processes carried out with methods different from the present invention are provided below.

The patent document numbered US4327115 relates to a method for the clarification and removal of hazes from fresh, pasteurized and fermented fruit juices by the use of "honey" as a clarifying agent, or alternatively "tannin/tannin derivatives and honey".

The patent document numbered WO2005070234A1 relates to use of cellulase and pectinolytic enzymes for the production of sediment free clarified fruit juices (banana, litchi and guava juice).

The patent document numbered USH89 relates to a process for removing turbidity from apple juices wherein the process comprises treating said juices with silica hydrogel and filtering through a diatomaceous earth filtering medium.

Patent applications pertaining to various studies performed with enzymes which will be used in the present invention are provided below.

The patent document numbered EP1763578 relates to coded polypeptides and nucleic acids having tannase activity and preparation thereof.

The patent document numbered US9394559 relates to a method for producing lactonic sophorolipids.

The patent document numbered CN107960569A relates to a method for preparing a citrus fruit vinegar beverage, specifically from ponkan (a type of citrus fruit), using a series of enzymatic and fermentation steps. This method focuses on producing a beverage that retains the health benefits of vinegar and the nutrients and flavors of ponkan. The process involves raw material treatment, alcohol fermentation, acetic acid fermentation, clarification, and seasoning treatment to produce a citrus fruit vinegar beverage that aims to improve taste and clarity compared to traditional methods.

The patent document numbered CN101491364B relates to a method for protecting the color of pomegranate juice and belongs to the field of deep processing of fruits. This method aims to maintain the original color of pomegranate juice after preparation and prevent it from fading over time. The process involves heating the pomegranate grains to a specific temperature before juicing, followed by cooling. Additionally, the method includes the addition of pectinase or amylase enzymes, followed by the use of clarifiers such as gelatin, bentonite, or silica sol, and finally concentrating under vacuum.

The article published by Cristina Garcia-Munoz and colleagues, "Metabolic Fate of Ellagitannins: Implications for Health, and Research Perspectives for Innovative Functional Foods, 2014," discusses the various health benefits associated with the dietary intake of ellagitannins (ETs). It is noted that ETs are not bioavailable as such and are metabolized in the body. In the upper gastrointestinal (GI) tract, ETs are partially converted to ellagic acid (EA), but this first metabolite is also poorly bioavailable. In the lower GI tract, EA and residual ETs are metabolized by gut microbiota to produce urolithins, which persist at relatively high concentrations in plasma and urine for days after dietary ET ingestion. Therefore, while ETs and EA may confer local health benefits on the GI tract, systemic health benefits are more likely to be attributed to urolithins. Cellular models suggest that urolithins are active against chronic degenerative diseases at physiological concentrations. Health benefits have been demonstrated in animal models and clinical studies. However, the critical role of gut microbiota in the bioconversion of ET leads to significant variability in physiological responses among humans, introducing the concept of high and low urolithin producers. This variability in obtaining potential health benefits from dietary ETs presents new challenges for the functional food industry. Different research perspectives are discussed to address this important issue for nutritionists, food technologists, and consumers.

An article published by Martina Cerreti et al (Optimization of pectinase and protease clarification treatment of pomegranate juice, 2017) focuses on clarification, a fundamental step in the pomegranate juice processing process, with the aim of improving the appearance and marketability of pomegranate juice. The aim of the study is to develop a customized pectinolytic and proteolytic enzymatic clarification process for pomegranate juice. Using response surface methodology, the effects of factors such as incubation time (30-120 min), temperature (25-50 °C) and amount of complex enzymes (0.1-0.4 g per 100 g of pomegranate juice) on the physical properties of pomegranate juice were analyzed. R2 and adjusted R2 values for cold haze, turbidity, potential haze, and clarity regression models are greater than 0.9 and 0.8, respectively. The amount of complex enzymes was the most important factor affecting the clarification of pomegranate juice. Additionally, increasing the amount of complex enzyme was found to cause protein loss and a significant decrease in phenol haze-forming activity. The optimum enzymatic treatment conditions are: temperature 25-30 °C, time 100-110 min, amount of protease-pectinase complex enzyme (protease:pectinase ratio 1:2) 0.22-0.25 g per 100 g of pomegranate juice. This study provides critical information for the optimization of the pomegranate juice clarification process and provides a basis for further development of this process.

An article published by A. Kapoor et al (Efficiency of Tannase Produced by Trichoderma Harzianum MTCC 10841 in Pomegranate Juice Clarification and Natural Tannin Degradation, 2013) found industrial applications in gallic acid production, beer, wine and fruit juice clarification and poultry feed detangration. It relates to a study on tannases (Tanin Acyl Hydrolase, E.C. 3.1.1.20), which are important enzymes. In this study, which was conducted to obtain industrially potent extracellular tannase, Trichoderma harzianum (MTCC no. 10841) was selected from a total of eighty-four fungal strains isolated for tannase production and optimization studies. Crude tannase has a maximum activity of 31.36 U/ml with 1.0% tannic acid substrate. The industrial potential of tannase obtained from Trichoderma harzianum on juice clarification and gallic acid production was investigated. A 57% reduction in tannin was observed in pomegranate juice treated with varying amounts of tannase at 40°C, and it was determined that this occurred without losing its biochemical properties such as pH, viscosity, sugar content and protein content. The natural tannin degradation efficiency of the enzyme was also investigated for gallic acid production using different tannin-rich agricultural residues. The enzyme showed maximum activity with amla fruits used as substrate and this was found to be higher than tannic acid. Thus, tannase from Trichoderma harzianum can be used for gallic acid production using cheaper agricultural residues.

### Technical Problems That the Present Invention Seeks to Solve

The present invention relates to the arrangement carried out in the clarification process of pomegranate juice. Accordingly, the clarification process will be carried out by applying the enzymatic method. The main purpose here is to enzymatically decompose hydrolyzed tannins and proteins in pomegranate juice.

As subrates of acridity, haze and sediment formation will be removed from fruit juice through enzymatic means, these defects of the pomegranate juice will be eliminated. Products which are obtained when hydrolyzed tannins and proteins are decomposed through enzymatic means will produce co-pigment effect, thereby increasing the stability and the color density of pomegranate juice.

### Description of the Invention

The present invention relates to a clarification process carried out to obtain a pomegranate juice that is clear, does not get hazy afterwards, does not create any sedimentation, and that has a color stable. In the invention, the enzymatic method is used as the clarification process. Tannese + lactonase + protease is used as enzyme. Therefore, the present invention relates to pomegranate fruit juice and fruit juice concentrate industry.

### Detailed Description of the Invention

Commercially available pomegranate juices are far from ensuring consumer satisfaction because of their high sediment content, acrid and bitter taste and brownish colors. Therefore, in our country, although being ranked as number three in world's gross pomegranate production, pomegranate constitutes only the 10% of fruits that are processed in fruit juice industry.

Food industry's problem of being unable to produce pomegranate juice having the desired taste and appearance is resolved and the production of a pomegranate juice that is preferred by consumers is achieved by means of the present invention. Initially, the general information is provided regarding the clarification process in pomegranate juices and subsequently, technical problems of available method are disclosed in order to provide a better understanding in terms of problems identified in the prior art.

Haze and sediment formation in fruit juices stem from microbial development and the presence of starch, pectin, polyphenol and protein. However, in cases where no microbial development is present, the main mechanism of action for haze and sediment formation, especially in fruit juices such as pomegranate juice in which the content of pectin and starch is substantially lower, is the reaction between polyphenols and proteins. However, the fact remains that only some of phenolic and protein groups contained in fruit juices exert influence on haze and sediment formation. In model systems created with proteins, studies have shown that simple phenols and many polyphenol monomers have actually no effect on sediment formation and the main polyphenol group that is effective on sediment formation is tannins. In that case, it will be possible to inhibit haze and sediment formation substantially by means of hydrolyzing tannins to simple phenols.

Pomegranate juices comprise 2 different tannin groups. These are hydrolysable tannins (gallotannins and ellagitannins) which are the dominant phenolic group in pomegranate juices and condensable tannins. Studies conducted within the context of the present invention indicated that condensable tannin amount (31-155 mg/L) is significantly lower when compared to hydrolysable tannin amount (1159 mg/L). Condensable tannins are identified as catechin and epicatechin polymers. Catechins and epicatechins have quite a mild influence on sediment formation, however, when their polymerization degree increases, they have the potential of exerting significant influence on sediment formation. In view of the fact that polymerization degrees of condensable tannins in pomegranate juices are quite low (varies between the range of 1.0-3.2) and that pomegranate juice contains such low quantities thereof, it can be understood that condensable tannins' influence on the sediment formation occurring in pomegranate juices is negligibly small. As a matter of fact, hydrolysable tannins (r=0.995) were demonstrated to be a lot more effective in terms of hazes in pomegranate juices when compared to condensable tannins (r=0.747) in preliminary studies conducted within the scope of the present invention. These results clearly indicated that along with proteins, substances that are mainly effective on haze and sediment formation in pomegranate juices are hydrolysable tannins.

Pomegranate juices comprise protein at a level of 7.17-10 g/L. However, what really exerts influence on sediment formation in fruit juices is the protein group present in the fruit juice, rather than the protein amount. In pomegranates, while globulin (43%) takes the first place with the highest amount, it is followed by albumin (32%), glutelin (16%) and prolamin (9%) respectively. As it is well established, prolamins are proteins containing high amounts of proline and glutamine. While proteins which do not contain proline in their structure do not get involved in sediment formation, protein's influence on sediment formation increases as the proline rate in the protein structure rises. Hence, based on the sediment formation occurring in pomegranate juices, it is quite clear that prolamin which is present in small quantities in pomegranate juices has an impact thereon. In consideration of the fact that free amino acids have no effect on haze, it is foreseen that hydrolyzing these proteins will be an important step towards preventing sediment formation and haze.

In addition to phenolic and protein types, these components' ratio to one another is also an important factor in terms of haze and sediment formation in fruit juices. Effects of ratio of tannin and protein amounts on sediment formation were investigated in model systems and accordingly, it was reported that sediment formation reached to a maximum when the protein amount was kept constant and the tannin amount was increased, and sediment formation decreased when the tannin amount was kept being increased. Analogously, an identical outcome was obtained when the tannin amount was kept constant and protein amount was increased.

In pomegranate juices, tannin amount is significantly higher when it is compared to the protein amount, accordingly, the entirety of active ends of proteins gets into a reaction with tannins. This results in formation of small flocks. Therefore, it takes time for these flocks to precipitate as sediment and accordingly, sediment formation is observed during storage period. This shows that removing one or two of substrates (hydrolysable tannin and/or proteins) that are required for sediment and haze formation by means of hydrolysis can solve the haze and sediment formation problem in pomegranate juices.

However, present applications have certain shortcomings. Existing clarification methods generally target phenolic agents contained in pomegranate juice. Whereas only some of phenolic groups have effects on haze and sediment formation. Simple phenols and monomeric phenols do not have any influence on haze and sediment formation. In clarification processes carried by using gelatin, albumin and casein, high rates of subsequent haze are observed even though there is a substantial reduction in total phenolic agent amount. Hence, haze and acridity problems cannot be resolved. Furthermore, these applications also cause removing a significant amount of anthocyanins which are the origin of the bright red color of the pomegranate juice. Contrarily, when xanthan gum and chitosan is used, hydrolyzed tannins are removed from the pomegranate juice at a much lower rate in comparison to other auxiliary clarification agents. Xanthan gum and chitosan exert influence on proteins, thereby providing clarification. They cause a reduction in the bioavailability of the fruit juice by removing proteins from pomegranate juice. By means of the present invention, however, a clarified pomegranate juice that is relieved of acridity and that does not form any subsequent sediment is produced and its bioavailability is also increased.

The present invention provides a clarification process that targerts compounds that cause defects in pomegranate juices. Hydrolysable tannins (gallotannin and ellagitannin) and proteins were subjected to enzymatic (tannase, lactonase and protease) hydrolysis. Gallotannins were hydrolyzed to gallic acid, while ellagitannins were hydrolyzed to hexahydroxydiphenic acid by using tannase. As it can be understood, when tannase enzyme is used solely, it can decompose gallotannins only to their constituents, and cannot hydrolyze ellagitannins completely. In view of the fact that ellagitannin amount (134-7507 mg/L) is much higher when compared to gallotannin amount (0-165 mg/L) in pomegranate juices, tannase activity by itself will not be enough for clarifying pomegranate juices. In the literature, only one study was found in which tannase is utilized for the purpose of clarifying pomegranate juices. In said study, effects of tannase activity on pH, titratable acidity, L* value, tannin and ascorbic acid amounts were investigated. As a result, it was determined that tannins were reduced by only 37% as tannase was used solely and clarification process could not have performed in adequate manner. In the present invention, however, lactonase and protease are utilized together with tannase. Hexahydroxydiphenic acid created as a result of the tannase activity was transformed into ellagic acid by means of the lactonase activity. Thus, tannins that are required for haze and sediment formation were enzymatically decomposed. Furthermore, by means of the protease activity, proteins were decomposed into amino acids which are known to have no effect on sediment formation. Eventually, no haze and sediment formation occurred since no substrate is present in the medium or they are present in very small amounts, and stability and color density of anthocyanins which provide the bright red color to pomegranate juices were improved through co-pigmentation effect by means of gallic acid, ellagic acid and amino acid. Additionally, sensory analysis tests have confirmed the fact that the main agent inducing acrid taste is actually hydrolysable tannins. Acridity emanates from the reaction between proteins existing in saliva and hydrolysable tannins. Thus, acrid taste of pomegranate juices is also reduced by means of the hydrolysis of hydrolysable tannins.

Differences of the present invention from the prior art is disclosed above. Briefly, existing methods do not directly target hydrolyzed tannins and proteins which are the main reasons of defects in pomegranate juices. These methods, without paying any specific attention to which group the phenolics belong, adopted the perceptive of inducing a reduction in phenolic agent amount would potentially eliminate defects in pomegranate juices.

In the present invention, differently from other methods, hydrolyzed tannins and proteins are targeted directly. Furthermore, in the present invention, instead of removing phenolics and proteins from the fruit juice by creating flocs or through filtration as in other methods, these are transformed into forms which will be directly used in human body. In the first process step, hydrolysable tannins were turned into gallic acid and ellagic acid by means of tannase and lactonase. In the second process step, proteins were turned into amino acids by means of protease.

Process steps of clarification process available in the state of the art and of the method according to the present are completely different from one another and these steps are disclosed below. Clarification process proposed in the present invention consists of two process steps. These are disclosed below:

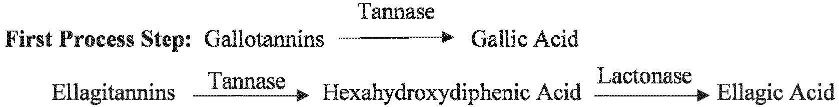

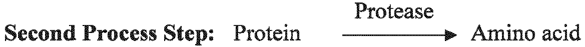

First process step comprises the hydrolysis of "hydrolysable tannins" which cause haze, sediment formation and acridity in pomegranate juices, through enzymes. Hydrolysable tannins are hydrolyzed by means of "tannase" and "lactonase" enzymes. Ellagitannins in pomegranate juices are hydrolyzed into ellagic acid, while gallotannins are hydrolyzed into gallic acid by using tannase and lactonase enzymes together. Since hydrolysable tannins which are one of the essential substrates for sediment formation, are hydrolyzed at the beginning, it is anticipated that an effective clarification will be ensured. It is determined that ellagic acid and gallic acid amounts in grape juices do not change even though high degrees of sediment formation is observed during the storage period. This shows that free ellagic acid and gallic acid do not have any effects on sediment formation. In that case, hydrolysable tannins' effects on haze and sediment formation are eliminated as a result of enzymatic hydrolysis. Moreover, acridity occurring due to the reaction between proteins in saliva and hydrolysable tannins is also eliminated through the enzymatic hydrolysis of hydrolysable tannins.

Second process step comprises the hydrolysis of "proteins" which cause haze and sediment formation in pomegranate juices, through enzymes. Proteins are hydrolyzed through protease in order to eliminate proteins effects on haze and sediment formation. Since amino acids which will be created as a result of this process, has no effect on sediment formation, proteins effect on haze and sediment formation are also eliminated by means of enzymatic hydrolysis. While effects of hydrolysable tannins and proteins which cause haze and sediment formation in pomegranate juice, are eliminated as a result of tannase, lactonase, and protease activities, color stability and density of the pomegranate juice are improved by means of the co-pigmentation of anthocyanins with gallic acid, ellagic acid and amino acids created as a result of the enzymatic hydrolysis without adding co-pigment to pomegranate juice for the first time.

In short term, this method can be utilized in the clarification of all fruit juices containing hydrolyzed tannin and protein including pomegranate juice. Eliminating aforementioned defects of pomegranate juice will increase the demand for pomegranate juice. Thus, production of a higher-quality and healthier pomegranate juice will be ensured.

The present invention can easily be adapted to available technologies. The present invention incurs no additional costs apart from using enzymes instead of auxiliary clarification agents used at present. Furthermore, the present invention renders husking process which is frequently used in the current state of pomegranate juice production, unnecessary. In that case, additional expense to be made by industrialist for husking equipment will be eliminated, thereby allowing to conserve the substantial amount of energy consumed for husking process. Therefore, in the proposed method, while only enzymes to be used for the first time incur additional expenditure, the present invention will allow for saving on both the cost of husking process equipment and on the energy to be consumed for husking process. Additionally, once the inventive clarification method is adopted by the industry, it will increase the demand for enzymes to be used in the method, thereby urging enzyme production companies to produce enzymes specific to clarification method and will result in a substantial decrease in enzyme prices. Besides, as stated in the literature, these enzymes can readily be produced out of microbial sources (by using *Aspergillus niger GH1, Penicillium variable, Lenties elegans* or *Lactobacillus plantarum MTCC 1407* for tannase, by using *Aspergillus niger, Penicillium cyanofulvium ATCC 10431* or *Fusarium oxysporum* for lactonase, and by using *Aspergillus niger* or *Penicillium sp.* for protease). For this reason, pomegranate juice producers will be able to produce these enzymes with their own laboratory means and accordingly, costs will decrease even more by putting the inventive clarification method into practice.

## Claims

1. A method for eliminating the defects acridity, haze and sediment formation in pomegranate juices and for ensuring color stability, **characterized in that** it includes the process steps of conversion of the pomegranate juices,
i. as the first process step, with tannase and lactonase enzymes
- converting gallotannins to gallic acids with tannase enzyme,
- converting ellagitannins to hexahydroxydiphenic acid with the tannase enzyme, converting this acid to ellagic acid with the lactonase enzyme
ii. as the second process step, converting proteins that cause problems into amino acids with the protease enzymes.

## Patentansprüche

1. Ein Verfahren zur Eliminierung der Defekte wie Schärfe, Trübung und Sedimentbildung in Granatapfelsäften und zur Gewährleistung der Farbstabilität, **dadurch gekennzeichnet, dass** es die Prozessschritte der Konvertierung der Granatapfelsäfte umfasst,
i. als erster Prozessschritt, mit den Enzymen Tannase und Lactonase
- die Konvertierung von Gallotaniinen in Gallensäuren mit dem Enzym Tannase,
- die Konvertierung von Ellagitanninen in Hexahydroxydiphensäure mit dem Enzym Tannase, Konvertierung dieser Säure in Ellagsäure mit dem Enzym Lactonase
ii. als zweiten Prozessschritt die Konvertierung von Proteinen, die zu Problemen führen, in Aminosäuren mit den Proteaseenzymen.

## Revendications

1. Méthode pour éliminer les défauts d'acridité, de brume et de formation de sédiments dans les jus de grenade et pour assurer la stabilité de la couleur, **caractérisée en ce qu'**elle comprend les étapes du processus de conversion des jus de grenade,
i. comme première étape du processus, avec les enzymes tannase et lactonase
- convertissant les gallotanniines en acides galliques avec l'enzyme tannase,
- convertissant les ellagitanins en acide hexahydroxydiphénique avec l'enzyme tannase, convertissant cet acide en acide ellagique avec l'enzyme lactonase
ii. comme deuxième étape du processus, convertissant les protéines qui causent des problèmes en acides aminés avec les enzymes protéasiques.
